# FASCICULE DE BREVET EUROPEEN

(11) **EP 2 081 547 B1**
(45) Date de publication et mention de la délivrance du brevet: **23.05.2012**
(21) Numéro de dépôt: 07848363.3
(22) Date de dépôt: 21.09.2007
(51) Int. Cl.: A61K 9/00, A61K 47/10

(54) **FORME GALENIQUE POUR L'ADMINISTRATION PAR VOIE TRANS-MUQUEUSE DE PRINCIPES ACTIFS**
GALENISCHE FORM ZUR TRANSMUKOSALEN VERABREICHUNG VON WIRKSTOFFEN
GALENIC FORM FOR THE TRANS-MUCOSAL DELIVERY OF ACTIVE INGREDIENTS

(30) Priorité: 22.09.2006 FR 0653899
(43) Date de publication de la demande: 29.07.2009
(73) Titulaire: Perovitch, Philippe, 33680 Le Temple (FR); Maury, Marc, 33160 Saint Medard en Jalles (FR)
(72) Inventeur: Perovitch, Philippe, 33680 Le Temple (FR); Maury, Marc, 33160 Saint Medard en Jalles (FR)
(74) Mandataire: CAPRI
(86) Numéro de dépôt international: PCT/FR2007/051993
(87) Numéro de publication internationale: WO 2008/035020

(56) Documents cités:
- WO-A-00/41692
- WO-A-01/15666
- GB-A- 2 291 593
- GB-A- 2 392 093

## Description

La présente invention concerne une forme galénique pour l'administration systémique instantanée par voie trans-muqueuse d'au moins un principe actif. L'invention se rapporte également à ses utilisations.

Actuellement, il existe un grand nombre d'actifs pharmaceutiques destinés à être auto-administrés par le patient lui-même. Le document WO 00/41692 décrit des compositions de ce type.

Cette auto-administration s'effectue généralement par la voie digestive.

Or, lorsqu'ils sont introduits dans le tube digestif et l'estomac, les principes actifs subissent l'effet dit de premier passage digestif, altérations et déperditions liées au milieu stomacal ou aux variations des physiologies intestinales. Ils sont ensuite soumis à un effet dit de « premier passage hépatique » qui provoque leur métabolisation et/ou leur dégradation plus ou moins intense, avec constitution de nombreux métabolites, pour la plupart inactifs ou toxiques.

La dose de principes actifs véritablement biodisponible est donc extrêmement faible : seule une part très résiduelle de la quantité administrée demeure valide pour produire l'effet pharmacologiquement attendu.

Ainsi, à titre d'exemple, administrées par voie digestive, certaines hormones sont détruites à plus de 80%. D'autres substances comme le Sumatriptan ont une biodisponibilité résiduelle réduite à moins de 14% de la dose administrée. On peut encore citer des opioïdes antalgiques qui ont une biodisponibilité qui varie entre 10 et 40%.

En outre ces principes actifs présentent généralement des temps de demi-vie très courts, inférieurs aux temps d'atteinte des pics plasmatiques.

On sait aussi que le début de l'efficacité thérapeutique pour le patient intervient au plus tôt 45 min après la prise, correspondant au délai d'absorption digestive, de métabolisation et de diffusion vasculaire, puis tissulaire et éventuellement intracellulaire.

De fait, il apparaît deux problèmes majeurs.

Le premier problème est qu'il faut administrer une dose suffisante au patient tenant compte de la dilution et de la dispersion dans l'organisme, pour que la partie significativement active qui atteint la zone affectée soit efficace.

Le second est le temps de latence dû à la métabolisation et à la diffusion dans l'organisme avant que la molécule agisse et que le patient en ressente les bienfaits.

On connaît une autre voie d'administration, la voie Per/Sublinguale, qui permet d'administrer des médicaments par franchissement passif des muqueuses sublinguales, jugales, gingivales, linguales, palatines ou pharyngées, puis passage dans les veines sub-linguales et distribution à la circulation générale, court-circuitant ainsi le passage digestif et le métabolisme hépatique.

Néanmoins, les formulations sublinguales existantes ne sont pas satisfaisantes, notamment en raison du fait que la plupart des médicaments sont instables et constitués de molécules par nature insolubles dans les liquides biologiques comme la salive. Elles sont souvent complexes et une grande partie des molécules reste généralement sous forme cristalline, rendant ainsi impossible l'absorption et donc le passage dans la circulation générale.

Il subsiste donc un besoin pour une formulation galénique permettant d'administrer une quantité immédiatement biodisponible de principe actif, de façon à pouvoir traiter très rapidement et efficacement des symptômes douloureux ou des troubles invalidants.

C'est ce à quoi répond la présente invention en proposant une forme galénique pour l'administration par voie trans-muqueuse d'au moins un principe actif en état de dissolution stable et complète, telle que décrite dans la revendication 1.

Par voie trans-muqueuse, on entend tout franchissement passif d'une molécule lipophile ou amphiphile à travers les muqueuses linguales, sublinguales, gingivales, palatines, jugales, ou tout autres muqueuses constitutives de la cavité buccale et de l'oropharynx.

Par état de dissolution stable et complète, on entend un état de dissolution restituant le principe actif à l'état moléculaire et faiblement ionisé dans son milieu de dissolution, état de dissolution prévenant toute éventualité d'une recristallisation inopportune.

Les principes actifs susceptibles d'être formulés selon l'invention sont des molécules solubles en mélanges hydroalcooliques. Ils sont préférentiellement de nature lipophile ou amphiphile et de faible poids moléculaire, inférieur à 10 000 Da.

De façon préférentielle, la forme galénique selon l'invention se présente sous forme d'une solution hydroalcoolique comprenant entre 20 et 95% d'alcool en masse et une teneur en eau comprise entre 5 et 80%. Le passage dans la circulation systémique du ou des principes actifs formulés selon l'invention, s'effectue donc en solution hydroalcoolique au degré variable d'alcool, préférentiellement compris entre 20 et 95°.

Selon une caractéristique majeure de l'invention, l'alcool, présent à au moins 20% en masse, ne joue pas seulement le rôle de solvant, mais également celui de promoteur d'une absorption per-muqueuse accélérée, dont la vitesse croit en fonction de l'élévation du degré d'alcool utilisé.

Selon un mode de réalisation préféré de l'invention, la solution hydroalcoolique est réalisée à base d'eau et d'éthanol.

La solution hydroalcoolique selon l'invention peut comprendre un tensioactif tel que le Crémophor ou un Polysorbate, un arôme ou un édulcorant pour adoucir la sensation gustative, mais seulement si cela s'avère indispensable car toute addition d'excipient(s) ampute le ratio de la dose absorbée en même temps qu'elle réduit la vitesse d'absorption per-muqueuse du ou des principes actifs considérés.

Selon un mode de réalisation particulier, lorsque les principes actifs à administrer contiennent une fonction acide carbonyle, la forme galénique selon l'invention peut aussi comprendre un agent correcteur de pH et/ou un agent séquestrant. En effet, les principes actifs contenant une fonction acide carbonyle peuvent réagir avec les alcools primaires et les alcools secondaires pour former un ester. Cette réaction conduit à la réduction de la teneur en principe actif et à l'apparition d'impuretés, ce qui est incompatible avec la préparation d'un médicament. L'adjonction d'au moins un agent correcteur de pH limite la concentration en ion acide H+ induisant la réaction d'estérification, et l'ad jonction d'au moins un agent séquestrant limite la concentration d'ions métalliques catalysant cette réaction, ce qui permet d'inhiber la formation d'esters des principes actifs contenant des ions carbonyles. Préférentiellement, l'agent correcteur de pH est choisi parmi les carbonates et bicarbonates de sodium, les phosphates monosodique ou disodique, la triéthanolamine, la soude (NaOH) et la potasse (KOH). L'agent séquestrant est choisi parmi l'acide éthylène-diamine-tétrnacétique (EDTA), le calcium disodium éthylène diamine tétra-acétate (E385), le glucono delta-lactone (E575), le sodium gluconate (E576), le potassium gluconate (E577) et le sodium tripolyphosphate.

La forme galénique selon l'invention permet aux principes actifs de franchir passivement les muqueuses de l'oropharynx dans un délai inférieur à 20 secondes après administration. Ce délai d'absorption très rapide permet de prévenir toute stagnation de la solution et du ou des principes actifs dans l'atmosphère buccale ainsi que leur mélange inopportun avec de la salive susceptible de les altérer, ce qui introduirait une rupture dans la continuité et la stabilité de la dissolution du ou des principes actifs. Ce court délai permet également de prévenir toute déglutition réflexe de la solution et du ou des principes actifs qu'elle contient. Le passage trans-muqueux d'un principe actif présenté en état de dissolution selon l'invention du côté de la membrane épithéliale externe, constituée de structures phospho-lipidiques qui absorbent passivement les molécules lipophiles par affinité élective, est basé sur un appel osmotique vers l'autre côté de ladite membrane, auquel participent ensemble la concentration en principe actif dissous et celle de la solution alcoolique considérée. L'appel osmotique est d'autant plus vivace et puissant que la molécule lipophile en état de dissolution est de faible poids moléculaire et que le degré d'alcool qui sert de promoteur d'absorption est élevé.

Les muqueuses de la bouche et de l'oropharynx possèdent un réseau de micro-vaisseaux très dense, quasi-spongieux, si bien que les molécules, tant de solvant alcoolique que de principe actif dissous, qui franchissent les pores lipophiles de la membrane épithéliale, sont instantanément capturées par la micro-circulation sanguine et collectées vers les veines sublinguales. Ce phénomène est accentué par la présence de l'alcool qui provoque une vasodilatation et un accroissement du débit micro-vasculaire local des muqueuses.

Du fait de ce débit circulatoire élevé localement, accru par l'alcool, il n'y a donc jamais d'équilibre de part et d'autre de la membrane : la concentration dans la bouche reste toujours plus importante, jusqu'à épuisement du mécanisme par défaut de molécules à absorber.

Ainsi, la totalité de l'alcool et du principe actif qui s'y trouve dissous selon l'invention, passe à travers la muqueuse.

L'utilisation de la forme galénique selon l'invention permet d'administrer une dose de principe actif qui est immédiatement absorbée dès que déposée au contact de la muqueuse, pour être distribuée dans l'instant par voie vasculaire à l'ensemble de l'organisme, sans aucun délai pour son action pharmacologique et sans subir les effets majeurs de passages digestifs et hépatiques.

La solution hydroalcoolique avec une teneur en éthanol d'au moins 20% en masse selon l'invention, présente également l'avantage de solubiliser des principes actifs même s'ils sont faiblement solubles, et de protéger la formulation pharmaceutique vis-à-vis d'une contamination microbiologique sans devoir introduire d'agent(s) de conservation anti-microbien(s).

Elle permet l'administration systémique instantanée à doses réduites et utiles de substances pharmacologiques telles que par exemple des hormones, des anti-hormones et toutes substances actives sur les glandes endocrines, des hypocholestérolémiants, des anti-infectieux, des anti-viraux, des anti-parasitaires, des anti-agrégants vasculaires, des molécules traitant la ménopause, l'andropause ou la stérilité, des contrnceptifs-minute, des antalgiques, des anti-inflammatoires, des substances anti-cnncéreuses_{;} des immunosuppresseurs, des anti-migraineux, des anti-émétiques, des anti-diarrhéiques, des anti-spasmodiques, des anti-allergiques, des anti-arythmiques, des érectogènes, des anxiolytiques, des nnti-diabétiques, des anti-hypertenseurs, des nnti-nsthmatiques, des anti-parkinsoniens et/ou des antihistaminiques.

En particulier, la forme galénique peut être utilisée pour la réalisation d'un médicament destiné au traitement et/ou à la prévention des crises migraineuses, des syndromes diarrhéiques, des crises allergiques, des vomissements, de la symptomatologie nauséeuse, des arythmies supra ventriculaires et ventriculaires, du syndrome de Raynaud, des troubles de l'érection, des dépressions, des troubles de panique, des troubles obsessionnels convulsifs (TOC), des anxiétés de type phobies sociales, des troubles du sommeil ou de l'éveil, du diabète, des affections pulmonaires et des crises d'asthme, des insuffisances hormonales cycliques et dysménorrhées, des crises douloureuses inflammatoires invalidantes, de la maladie de Parkinson, ou des affections neuro-dégénératives.

Avantageusement, la présente invention offre une grande simplicité de réalisation et une très bonne stabilité galénique : le réglage spécifique pour chaque molécule de la solution eau/ethanol garantit la solubilisation du principe actif tout en supprimant la plupart des excipients utilisés dans les formes pharmaceutiques usuelles et les formes sublinguales traditionnelles. Elle permet donc à la fois de réduire les coûts de fabrication et de diminuer les risques d'intolérance et les possibles interactions entre principe(s) actif(s) et excipients. De façon singulière, les délais d'action sont très courts, en particulier comparés aux lenteurs d'absorptions des médicaments par voie digestive. La délivrance pharmacologique quasi-instantanée permet à un patient de s'administrer lui-même un produit pour un effet presque équivalent à l'efficacité d'une injection intraveineuse en flash dans la circulation.

En outre, le principe actif ne rencontrant pas d'obstacle significatif pour son assimilation et sa distribution instantanée dans l'organisme, la dose de base administrée est très faible, la plus proche de la dose utile pour exercer l'activité pharmacologique requise. Cette dose est préférentiellement inférieure à 300 mg de principe actif.

Par ailleurs, la muqueuse oropharyngée disposant d'une surface totale d'absorption extrêmement large, démultipliée par son caractère de tissu villeux plissé, l'administration de la forme galénique selon l'invention est dépourvue d'un quelconque risque de déglutition intempestive ou de fausse route. En effet, elle permet un passage per-muqueux extrêmement rapide qui prévient toute dissolution salivaire ou déglutition des principes actifs administrés, avec l'avantage de pas déstabiliser les muqueuses, avec des dérivés tensioactifs par exemple, comme c'est le cas des formulations existantes.

De même, les effets de l'alcool sont insignifiants. A titre d'exemple, 2 à 4 ml d'Ethanol à 40°C ne sauraient produire qu'une alcoolémie circulante inférieure à 8 ou 16 mg par litre de sang, soit, par exemple, 31,25 à 62,5 fois en dessous des tolérances légales françaises de 0,5g d'alcool par litre de sang.

Selon un aspect de l'invention, la forme galénique nécessite un conditionnement industriel spécifique, afin de prévenir la dégradation du ou des principes actifs au contact de l'air.

Un mode de réalisation particulier consiste à utiliser un conditionnement métalloplastique souple opaque rempli sous atmosphère d'azote, pour la protection de la stabilité de la composition et l'imperméabilité à l'oxygène et aux rayonnements. Ce conditionnement garantit la stabilité dans le temps des principes actifs dissous en solution hydroalcoolique selon l'invention.

Pour le confort d'utilisation par le patient, pour un transport aisé, on peut préférentiellement recourir à des emballages "stick" sous forme d'étuis étanches spécifiques unidoses ou multidoses d'une contenance maximum de 5ml qui est le volume d'administration. Encore plus préférentiellement, la forme galénique selon l'invention est conditionnée dans des sticks unidoses de 0,25 à 5 ml, susceptibles de fournir une dose adéquate de principes actif.

De façon avantageuse, ce conditionnement est facile à transporter et permet une utilisation aisée de la forme galénique à tout moment de la journée. D'autres caractéristiques et avantages ressortiront des exemples qui vont suivre de l'invention.

### I. Exemple d'application au SUMATRIPTAN

Le Sumatriptan est l'anti-migrnineux majeur et de référence du marché. Toutefois, cette molécule de la famille des agonistes de la Sérotonine, lorsqu'elle est administrée par la voie entérale, possède un délai d'action d'environ deux heures, démesuré par rapport à l'attente de tout patient en souffrance, et une biodisponibilité dérisoire.

Il est possible d'utiliser la forme galénique selon l'invention pour l'administration du Sumatriptan. Une telle administration permet de produire une efficacité au niveau cérébral dans un délai de quelques minutes seulement pour un dosage compris par exemple entre 5 et 10 mg de Sumatriptan base.

On peut citer deux exemples de formulation du Sumatriptan selon l'invention.

### 1 - Exemple de formulation 1ml pour 5mg de Sumatriptan :

- eau distillée : 0,65ml
- éthanol, alcool absolu: 0,35ml
- Sumatriptan base : 5,0mg
- Edulcorant, Saccharinate de Sodium 0,3mg
et/ou Arôme qsp.

### 2 - Exemple de formulation 2ml pour 10mg de Sumatriptan :

- eau distillée : 1,30ml
- éthanol, alcool absolu : 0,70ml
- Sumatriptan base : 10,0mg
- Edulcorant, Saccharinate de Sodium 0,5mg
et/ou Arôme qsp.

### 3 - Exemple de formulation 0,75ml pour 6mg de Sumatriptan :

- eau distillée : 0,45ml
- éthanol, alcool absolu: 0,30ml
- Sumatriptan base: 6,0mg
- Edulcorant, Saccharinate de Sodium 0,5mg
   et/ou Arôme qsp.

Les formulations selon l'invention montrent une bonne stabilité dans le temps.

Le tableau ci-dessous présente des résultats d'études de stabilité à 3 mois, menées sur l'exemple de formulation 3 :

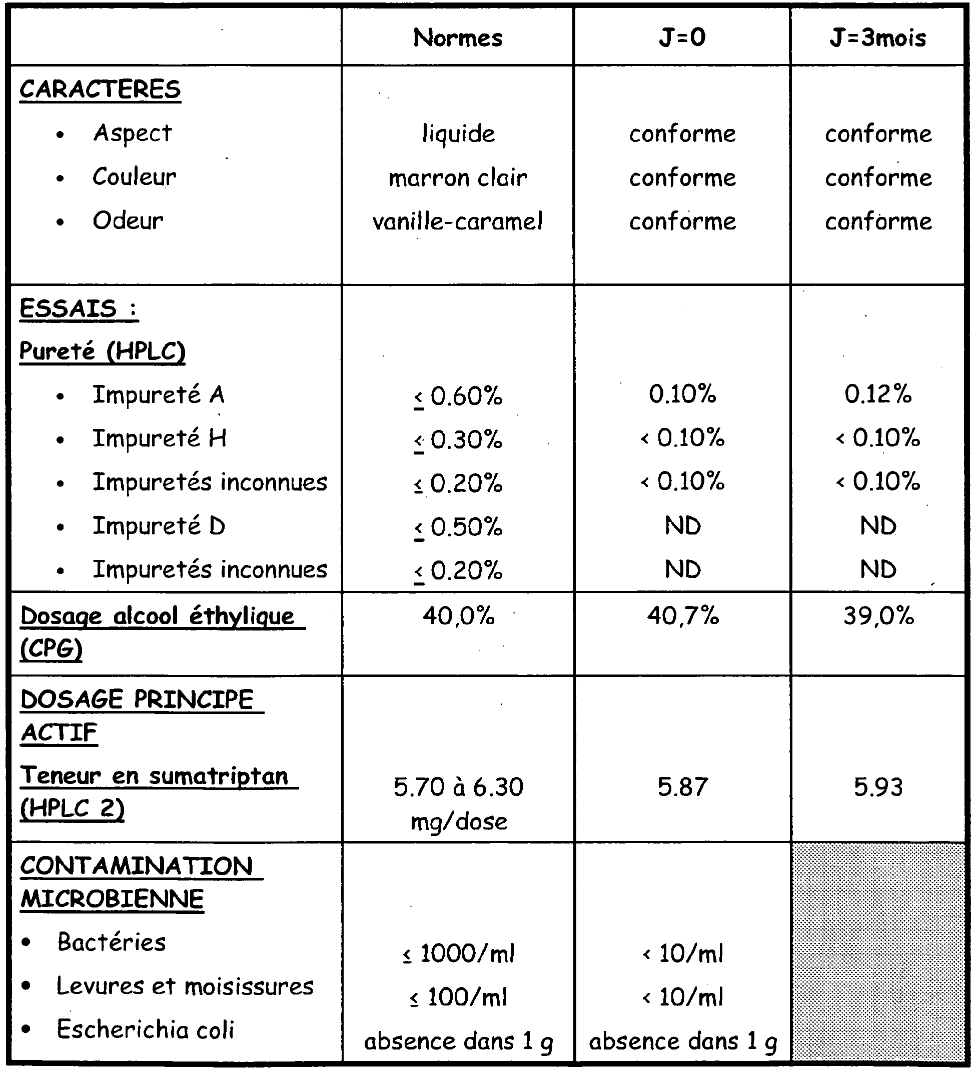

### II. Exemple d'application au LOPERAMIDE

Le Lopéramide est un anti-diarrhéique, anti-spasmodique et antalgique digestif synthétique de type opioïde.

Cette molécule est généralement dosée à 2mg et présentée sous forme gélule ou solution buvable.

Or, lorsqu'il est administré par voie digestive, le Lopéramide subit un premier passage digestif qui ne laisse disponible que 40% de la dose administrée, puis il subit une importante métabolisation hépatique. Le pic plasmatique n'est atteint qu'au bout de deux heures et la part de la dose effectivement biodisponible ne serait pas supérieure à 0,25mg sur les 2mg administrés.

Il est possible d'utiliser la forme galénique selon l'invention pour l'administration du Lopéramide. Une telle administration permet d'offrir un soulagement quasi-immédiat au patient dont les syndromes douloureux et diarrhéiques sont réduits significativement.

On peut citer deux exemples de formulation du Lopéramide selon l'invention.

### 1 - Exemple de formulation 0,5ml pour 0,25mg de Lopéramide :

- eau distillée : 0,35ml
- éthanol, alcool absolu : 0,15ml
- Lopéramide base : 0,25mg
- Edulcorant, Saccharinate de Sodium 0,20mg
et/ou Arôme qsp.

### 2 - Exemple de formulation 1ml pour 0,50mg de Lopéramide :

- eau distillée : 0,70ml
- éthanol, alcool absolu : 0,30ml
- Lopéramide base : 0,50mg
- Edulcorant, Saccharinate de Sodium 0,30mg
et/ou Arôme qsp.

### III. Exemple d'application aux antiallergiques antihistaminiques : LORATADINE et CETIRIZINE

Les antiallergiques antihistaminiques sont des médicaments dont le but est d'améliorer le confort des sujets allergiques. Ils agissent spécifiquement sur une molécule qui intervient de façon prépondérante dans les mécanismes d'inflammation et d'allergie : l'histamine.

Parmi les antiallergiques antihistaminiques non sédatifs couramment utilisés, on peut citer la Loratadine et la Cétirizine. Or, pour l'administration de 10mg de ces molécules par voie digestive, les pics plasmatiques ne sont atteints qu'au bout d'une heure, ce qui n'en fait pas un traitement approprié de la crise allergique.

Il est possible d'utiliser la forme galénique selon l'invention pour l'administration de la Loratadine ou de la Cétirizine. Une telle administration permet de réduire efficacement l'intensité des crises dans un temps assez court, inférieur à 15 minutes, qui correspond au passage vasculaire du principe actif puis à sa distribution périphérique au niveau tissulaire pour bloquer les récepteurs histamino-dépendnnts.

De plus, la réaction allergique s'accompagnant toujours d'une hypervascularisation locale, la forme galénique selon l'invention permet un accès facilité des principes actifs aux territoires affectés.

On peut citer trois exemples de formulation de la Cétirizine selon l'invention, et trois exemples de formulation de la Loratadine selon l'invention.

### 1 - Exemple de formulation 0,5ml pour 1mg de Cétirizine :

- eau distillée : 0,365ml
- éthanol, alcool absolu : 0,135ml
- Cétirizine base : 1,0mg
- Edulcorant, Saccharinate de Sodium 0,2mg
et/ou Arôme qsp.

### 2 - Exemple de formulation 1ml pour 3mg de Cétirizine:

- eau distillée : 0,675ml
- éthanol, alcool absolu : 0,325ml
- Cétirizine base : 3,0mg
- Edulcorant, Saccharinate de Sodium 0,40mg
et/ou Arôme qsp.

### 3 - Exemple de formulation 1ml pour 5mg de Cétirizine :

- eau distillée : 0,575ml
- éthanol, alcool absolu : 0,425ml
- Cétirizine base : 5,0mg
- Edulcorant, Saccharinate de Sodium 0,6mg
et/ou Arôme qsp.

### 4 - Exemple de formulation 0,5ml pour 1mg de Loratadine :

- eau distillée : 0,365ml
- éthanol, alcool absolu : 0,135ml
- Loratadine base : 1,0mg
- Edulcorant, Saccharinate de Sodium 0,2mg
et/ou Arôme qsp.

### 5 - Exemple de formulation 1ml pour 3mg de Loratadine :

- eau distillée : 0,675ml
- éthanol, alcool absolu : 0,325ml
- Loratadine base : 3,0mg
- Edulcorant, Saccharinate de Sodium 0,4mg
et/ou Arôme qsp.

### 6 - Exemple de formulation 1,5ml pour 5mg de Loratadine :

- eau distillée : 1,075ml
- éthanol, alcool absolu : 0,425ml
- Loratadine bnse : 5,0mg
- Edulcorant, Saccharinate de Sodium 0,6mg
et/ou Arôme qsp.

### IV. Exemple d'application aux anti-émétigiues : METOPIMAZINE et DOMPERIDONE

Les anti-émétiques, comme la Métopimazine ou le Dompéridone, sont des médicaments couramment utilisés pour traiter les crises de vomissements.

Ils sont généralement administrés sous des dosages compris entre 10 et 15 mg, sous formes de comprimés, lyocs, sirops ou solutions buvables.

Or ces formes d'administration présentent toutes une inadéquation thérapeutique, car les molécules sont très souvent rejetées par les mêmes vomissements qu'elles doivent traiter, avant d'avoir eu un quelconque effet.

Il est possible d'utiliser la forme galénique selon l'invention pour l'administration de la Métopimazine ou du Dompéridone. Une telle administration évite le problème du rejet de l'actif par vomissement et permet un effet thérapeutique quasi-immédiat.

On peut citer un exemple de formulation du Métopimazine selon l'invention, et un exemple de formulation du Dompéridone selon l'invention.

### 1- Exemple de formulation 1ml pour 2mg de Métopimazine :

- eau distillée : 0,675ml
- éthanol, alcool absolu : 0,325ml
- Métopimazine base : 2,0mg
- Edulcorant, Saccharinate de Sodium 0,2mg
et/ou Arôme qsp.

### 2 - Exemple de formulation 1ml pour 2mg de Dompéridone :

- eau distillée : 0,550ml
- éthanol, alcool absolu : 0,450ml
- Dompéridone base : 2,0mg
- Edulcorant, Saccharinate de Sodium 0,20mg
et/ou Arôme qsp.

### V. Exemple d'application aux anti-nnuséeux : DIMENHYDRINATE et DIPHENHYDRAMINE

Parmi les anti-nauséeux disponibles sur le marché, on connaît notamment le Dimenhydrinate et la Diphenhydramine.

Ces molécules administrées par voie digestive présentent une forte métabolisation hépatique et un délai d'action retardé par rapport à la prise, avec un mauvais rendement dose/efficacité.

Il est possible d'utiliser la forme galénique selon l'invention pour l'administration du Dimenhydrinate ou de la Diphenhydramine. Une telle administration permet une biodisponibilité immédiate de faibles doses actives et un soulagement rapide de la symptomatologie nauséeuse.

On peut citer un exemple de formulation du Dimenhydrinate selon l'invention, et un exemple de formulation de Diphenhydramine selon l'invention.

### 1 - Exemple de formulation 1ml pour 5mg de Dimenhydrinate :

- eau distillée : 0,650ml
- éthanol, alcool absolu 0,350ml
- Dimenhydrinate base : 5,0mg
- Edulcorant, Saccharinate de Sodium 0,3mg
et/ou Arôme qsp.

### 2 - Exemple de formulation 1,5ml pour 8mg de Diphenhydramine :

- eau distillée : 0,900ml
- ethanol, alcool absolu : 0,600ml
- biphenyhydramine base : 8,0mg
- Edulcorant, Saccharinate de Sodium 0,6mg
et/ou Arôme qsp.

### VI. Exemple d'application aux anti-arythmiques

Actuellement, il existe deux anti-arythmiques majeurs, l'Amiodarone et le Flécaïnide.

L'Amiodarone est connue pour traiter et prévenir les arythmies supraventriculaires et ventriculaires que ce soient les tachycardies supraventriculaires, les fibrillations auriculaires, les tachycardies arythmiques du syndrome de Wolff Parkinson White, les arythmies ventriculaires sévères ou encore les tachycardies ventriculaires et fibrillations ventriculaires. L'Amiodarone est aussi indiquée dans la réanimation cardio-respiratoire en cas d'arrêt cardiaque lié à une fibrillation ventriculaire résistante aux chocs électriques externes.

Cette application thérapeutique ancienne, est efficace mais productrice d'effets secondaires importants et invalidants, liés à l'excès d'iode ingéré puis libéré pour obtenir des concentrations thérapeutiques.

En effet, pour que l'Amiodarone soit biodisponible, il faut que l'organisme entier en soit saturé afin qu'il la relargue ensuite en continuité.

Le traitement d'urgence est problématique car il faut administrer des doses élevées, l'action pharmacologique est très lente, et l'usage est limité à une administration en milieu hospitalier pour pouvoir surveiller les effets secondaires liés à la prise.

Il est possible d'utiliser la forme galénique selon l'invention pour l'administration de l'Amiodarone. Une telle administration permet de fournir une dose active immédiatement absorbée par l'endocarde, zone tissulaire et organique du coeur, qui accapare de manière privilégiée l'Amiodarone par le mécanisme dit d'endocytose au seul contact du sang circulant porteur d'Amiodarone, pour une quantité limitée circulant dans le réseau vasculaire.

Le ratio dose/effet est très bon et peut permettre un traitement facilité et rapide des crises sans hospitalisation.

L'invention offre donc la possibilité d'administrer des doses moindres, modulables dans le temps, adaptées au statut pathologique et au niveau de réponse thérapeutique de chaque patient.

On peut citer un exemple de formulation de l'Amiodarone selon l'invention.

### Exemple de formulation 2ml pour 25mg d'Amiodarone:

- eau distillée : 1ml
- éthanol, alcool absolu : 1ml
- Amiodarone base : 25,0mg
- Edulcorant, Saccharinate de Sodium 0,6mg
et/ou Arôme qsp.

### VII. Exemple d'application aux hormones

Toutes les hormones administrées par voie digestive, subissent des dégradations dans le tube digestif puis un hypermétabolisme hépatique de premier passage qui à la fois dégrade leurs structures stéréochimiques et fabrique des métabolites capables de générer des effets secondaires. Leur biodisponibilité .résiduelle est donc souvent très faible.

Il est possible d'utiliser la forme galénique selon l'invention pour l'administration d'hormones. Une telle administration permet une délivrance et une fixation sans délai aux récepteurs hormonaux tissulaires, pour une activité pharmacologique efficace avec une forte diminution des effets secondaires.

Parmi les différentes hormones utilisées en hormonothérapies, on peut citer la DHEA (DéHydroEpiAndrostérone). Cette hormone est fabriquée par l'organisme pour servir de précurseur aux oestrogènes et aux androgènes. Son taux de fabrication diminue fortement avec l'âge.

Lorsqu'elle est administrée par voie digestive, la DHEA subit un métabolisme hépatique considérable produisant des métabolites générateurs d'effets secondaires androgéniques et même cancérigènes.

La présente invention permet une administration de la dose nécessaire de DHEA pour compenser le déficit hormonal lié à l'âge, sans risque d'effets secondaires. On peut citer un exemple de formulations du DHEA selon l'invention.

### Exemple de formulation 2ml pour 10mg de DHEA :

- eau distillée : 1,2ml
- éthanol, alcool absolu 0,8ml
- DHEA base : 10,0mg
- Edulcorant, Saccharinate de Sodium 0,6mg
et/ou Arôme qsp.

Parmi les hormones connues, on peut également citer l'Estradiol, hormone oestrogénique, actuellement administrée par voie per-muqueuse nasale, avec une biodisponibilité moyenne réduite, inférieure à 25% de la dose administrée.

Il est passible d'utiliser la forme galénique selon l'invention, à distribution vasculaire et tissulaire rapide, pour administrer de l'Estradiol. Avantageusement, une telle administration permet de compenser les troubles parfois invalidants et survenant brutalement, liés au périodes de déficit du taux d'oestrogènes.

### Exemple de formulation 0,5ml pour 100µg d'Estradiol :

- eau distillée : 0,3ml
- éthanol, alcool absolu : 0,2ml
- estradiol base : 100µg
- Edulcorant, Saccharinate de Sodium 0,1mg
et/ou Arôme qsp.

### VIII. Exemple d'application aux anti-inflammatoires, antalgiques

Parmi les anti-inflammatoires antalgiques couramment utilisés, on connaît notamment l'Ibuprofène, le Kétoprofène et le Diclofénac.

Ces molécules, lorsqu'elles sont administrées par voie digestive, ont un délai d'action supérieur à une heure et demi.

Il est possible d'utiliser la forme galénique selon l'invention pour l'administration de ces anti-inflammatoires antalgiques. Une telle administration permet de soulager très rapidement des douleurs diversifiées qu'elles soient articulaires, traumatiques voire migraineuses.

On peut citer un exemple de formulation du Diclofénac selon l'invention. Exemple de formulation 1ml pour 10mg de Diclofénac:
- eau distillée : 0,55ml
- éthanol, alcool absolu : 0,45ml
- Diclofénac base : 10,0mg
- Edulcorant, Saccharinate de Sodium 0,5mg
et/ou Arôme qsp.

### IX. Exemple d'application aux érectogènes

Trois produits dominent le marché des érectogènes : le Sildénafil, le Vardénafil et le Tadalafil.

Ces molécules présentent une biodisponibilité par voie orale moindre du fait d'un très important premier passage hépatique. L'effet pharmacologique et le pic plasmatique de ces molécules demandent un délai de 30 à 120 minutes, ce qui implique une prise anticipée par rapport au moment où l'effet érectogène est escompté.

Il est possible d'utiliser la forme galénique selon l'invention pour l'administration de ces érectogènes. Une telle administration permet une biodisponibilité immédiate du principe actif et donc un effet érectogène très rapide de l'ordre de quelques minutes.

On peut citer deux exemples de formulation du Sildénafil selon l'invention, et deux exemples de formulation du Tadalafil selon l'invention.

### 1 - Exemple de formulation 1ml pour 10mg de Sildénafil:

- eau distillée : 0,55ml
- éthanol, alcool absolu 0,45ml
- Sildénafil base : 10,0mg
- Edulcorant, Saccharinate de Sodium 0,5mg
et/ou Arôme qsp.

### 2 - Exemple de formulation 1,5ml pour 20mg de Sildénafil:

- eau distillée : 0,825ml
- éthanol, alcool absolu : 0,675ml
- Sildénafil base : 20,0mg
- Edulcorant, Saccharinate de Sodium 0,6mg
et/ou Arôme qsp.

### 3 - Exemple de formulation 0,5ml pour 2mg de Tadalafil :

- eau distillée : 0,375ml
- éthanol, alcool absolu : 0,225ml
- Tadalafil base : 2,0mg
- Edulcorant, Saccharinate de Sodium 0,2mg
et/ou Arôme qsp.

### 4 - Exemple de formulation 1ml pour 5mg de Tadalafil:

- eau distillée : 0,55ml
- éthanol, alcool absolu : 0,45ml
- Tadalafil base : 5,0mg
- Edulcorant, Saccharinate de Sodium 0,4mg
et/ou Arôme qsp.

### X. Exemple d'application aux anti-asthmatiques

Les traitements actuels de l'asthme par aérosolthérapies sont efficaces pour assurer l'entretien d'une bronchodilatation médiane équilibrée, mais dans le cas d'un bronchospasme massif et d'installation rapide, ils s'avèrent totalement inefficaces.

En effet, plus de la moitié des anti-asthmatiques, comme le Salbutamol, le Salmétérol, le (R) Levosalbutamol, etc., administrés en aérosols inhalés ne dépassent pas le carrefour oro-pharyngé ou sont absorbés par l'oesophage, De plus, dans le cas d'une crise d'asthme, le blocage inspiratoire et le niveau périphérique du bronchospasme font que les traitements inhalés sont défaillants. Une crise d'asthme brutale et massive nécessite donc une intervention en urgence et une injection d'anti-asthmatiques par voie sous-cutanée.

Il est possible d'utiliser la forme galénique selon l'invention pour l'administration d'anti-asthmatiques. Une telle administration permet au patient d'être autonome pour faire face à l'urgence et lever rapidement la crise par ses propres moyens, par une voie aisée et rapide ne requérant qu'un simple geste.

Les molécules administrées selon l'invention atteignent immédiatement le coeur droit pour être aussitôt distribuées par les artères pulmonaires à l'ensemble des tissus bronchiques induisant la levée instantanée du bronchospasme.

On peut citer deux exemples de formulation du Salbutamol selon l'invention.

### 1- Exemple de formulation 0,5ml pour 0,25mg de Salbutamol:

- eau distillée : 0,275ml
- éthanol, alcool absolu : 0,225ml
- Salbutamol base : 0,25mg
- Edulcorant, Saccharinate de Sodium 0,10mg et/ou Arôme qsp.
- Acide chlorhydrique ou Acide sulfurique,
qsp ajuster le pH entre 2 et 5.

### 2 - Exemple de formulation 1ml pour 0,5mg de Salbutamol :

- eau distillée : 0,575ml
- éthanol, alcool absolu : 0,425ml
- Salbutamol base : 0,50mg
- Edulcorant, Saccharinate de Sodium 0,3mg et/ou Arôme qsp.
- Acide chlorhydrique ou Acide sulfurique,
qsp ajuster le pH entre 2 et 5.

On peut citer deux exemples de formulation de (R) Levosalbutamol selon l'invention.

### 1 - Exemple de formulation 0,5ml pour 0,25 mg de (R) Levosalbutamol :

- eau purifiée : qsp. 0,5ml
- éthanol, alcool absolu: 0,15ml
- (R) Levosalbutamol base : 0,1g
- Acide chlorhydrique, qsp ajuster le pH entre 2 et 5.

### 2 - Exemple de formulation 0,5ml pour 0,5mq de (R) Levosalbutamol :

- eau purifiée : qsp. 0,5ml
- éthanol, alcool absolu : 0,15ml
- (R) Levosalbutamol : 0,5mg
- Acide sulfurique, qsp ajuster le pH entre 2 et 5.
- Arôme qsp.

### XI. Exemple d'application aux anti-pnrkinsoniens : APOMORPHINE et SELEGILINE

La maladie de Parkinson est une maladie du système nerveux extrapyramidal qui se caractérise par la triade " tremblement, rigidité, akinésie ".

Le traitement est généralement la L-DOPA associée à un inhibiteur de la Décarboxylase. Ce traitement donne lieu à des échappements pharmacologiques, appelés effets « On/Off ». Lors de ces effets, le patient se trouve en situation de réapparition de sa dyskinésie avec une perte significative d'autonomie.

II existe actuellement un moyen de rompre ces épisodes par injection sous-cutanée d'Apomorphine Chlorhydrate avec un délai d'action entre 2 et 10 minutes et une durée d'action entre 45 et 90 minutes.

Par voie orale, l'Apomorphine est presque totalement métabolisée par le foie, avec une biodisponibilité entre 1 et 2% de la dose, tous les métabolites étant inactifs.

Il est possible d'utiliser la forme galénique selon l'invention pour l'administration d'Apomorphine. Une telle administration permet au patient parkinsonien de rompre ses états « On/Off », en autonomie, dans un délai d'action au moins aussi court que la prise sous-cutanée.

Par ailleurs, pour lutter contre les symptômes de la maladie de Parkinson; il existe aussi la Sélégiline utilisée en monothérapie ou en soutien de la L-DOPA thérapie. Cette molécule, agoniste de la dopamine, présente une très brève et faible biodisponibilité absolue par voie orale.

L'utilisation de la forme galénique selon l'invention pour l'administration de la Sélégiline permet une absorption instantanée avec une activité pharmacologique quasi-immédiate et complète.

On peut citer deux exemples de formulation de l'Apomorphine selon l'invention, et deux exemples de formulation de la Sélégiline selon l'invention.

### 1 - Exemple de formulation 0,5ml pour 3mg d'Apomorphine:

- eau distillée : 0,275ml
- éthanol, alcool absolu : 0,225ml
- Apomorphine base : 3,0mg
- Edulcorant, Saccharinate de Sodium 0,2mg
et/ou Arôme qsp.

### 2 - Exemple de formulation 1 ml pour 5mg de Apomorphine :

- eau distillée : 0,550ml
- éthanol, alcool absolu : 0,450ml
- Apomorphine base : 5,0mg
- Bisulfite de sodium : 0,5mg
- Edulcorant, Saccharinate de Sodium 0,4mg
et/ou Arôme qsp.

### 3 - Exemple de formulation 0,5mlpour 0,25mg de Sélégiline

- eau distillée : 0,275ml
- éthanol, alcool absolu : 0,225ml
- Sélégiline base : 0,25mg
- Bisulfite de sodium : 0,2mg
- Edulcorant, Saccharinate de Sodium 0,2mg
et/ou Arôme qsp.

### 4 - Exemple de formulation 1ml pour 0,5mg de Sélégiline

- eau distillée : 0,275ml
- éthanol, alcool absolu : 0,225ml
- Sélégiline base : 0,5mg
- Bisulfite de sodium : 0,4mg
- Edulcorant, Saccharinate de Sodium 0,2mg
et/ou Arôme qsp.

Bien entendu, l'invention n'est évidemment pas limitée aux exemples représentés et décrits ci-dessus, mais couvre au contraire toutes les variantes.

## Revendications

1. Forme galénique pour son utilisation en tant que médicament dans l'administration par voie trans-muqueuse d'au moins un principe actif, **caractérisée en ce qu'**elle comprend comme principe actif le sumatriptan, le lopéramide, la cétirizine, la loratadine, la métopimazine, le dompéridone, le dimenhydrinate, la diphenhydramine, l'amiodarone, le flécaïnide, la DHEA, l'estradiol, l'ibuprofène, le kétoprofène, le diclofénac, le sildénafil, le vardénafil, le tadalfil, le salbutamol, le salmétérol, le (R) lévosalbutamol, l'apomorphine, ou la sélégiline, ledit principe actif étant en état de dissolution stable et complète dans une solution hydro-alcoolique à base d'eau et d'éthanol, comprenant entre 20% et 95% d'éthanol et entre 5% et 80% d'eau en masse, de façon à permettre une absorption rapide dudit principe actif à travers les muqueuses sublinguales, gingivales et/ ou jugales.

2. Forme galénique selon la revendication 1, dans laquelle le au moins un principe actif a un poids moléculaire inférieur à 10 000 Da.

3. Forme galénique selon l'une des précédentes revendications, dans laquelle elle est conditionnée au moyen d'un contenant souple, garantissant la stabilité du ou des principe(s) actif(s) dans leur dissolution dans le temps.

4. Forme galénique selon la revendication 3, dans laquelle le contenant se présente sous forme d'un étui étanche unidose ou multidoses d'une contenance maximum de 5ml.

5. Forme galénique selon l'une quelconque des revendications précédentes, comprenant en outre un agent correcteur de pH et/ou agent séquestrant si ledit principe actif contient une fonction carbonyle.

6. Utilisation de la forme galénique selon l'une des revendications 1 à 5, pour la réalisation d'un médicament destiné au traitement et/ou à la prévention des crises migraineuses, des syndromes diarrhéiques, des crises allergiques, des vomissements, de la symptomatologie nauséeuse, des arythmies supra ventriculaires et ventriculaires, des troubles de l'érection, des affections pulmonaires et des crises d'asthme, des insuffisances hormonales cycliques et dysménorrhées, des crises douloureuses inflammatoires invalidantes, de la maladie de Parkinson.

## Claims

1. Galenical form for it use as a drug with the administration by transmucous way of at least one active ingredient, **characterized in that** said active ingredient comprises sumatriptan, loperamide, cetirizine, loratadine, metopimazine, domperidone, dimenhydrinate, diphenhydramine, amiodarone, flecaïnide, DHEA, estradiol, ibuprofene, ketoprofene, diclofenac, sildenafil, vardenafil, tadalfil, salbutamol, salmeterol, (R) levosalbutamol, apomorphine or selegiline, said active ingredient being in a stable and complete dissolved state in a hydroalcoholic solution of water and ethanol that comprises between 20% and 95% of ethanol and between 5% and 80% by mass of water, so as to allow rapid absorption of said active ingredient through the sublingual, gingival and/or jugal mucous.

2. Galenical form according to claim 1, wherein the at least one active ingredient has a molecular weight that is less than 10,000 Da.

3. Galenical form according to one of the preceding claims, wherein it is packaged by means of a flexible container, ensuring the stability of the active ingredient(s) in its/their dissolving over time.

4. Galenical form according to claim 3, wherein the container comes in the form of a single-dose or multidose airtight case with a maximum capacity of 5 ml.

5. Galenical form according to one of the preceding claims, wherein the hydroalcoholic solution comprises a pH-correcting agent and/or a sequestering agent if said active ingredient contains a carbonyl function.

6. Use of the galenical form according to one of claims 1 to 5 for the production of a drug that is intended for treatment and/or for preventing migraine attacks, diarrhea syndromes, allergy attacks, vomiting, nauseous symptomatology, supraventricular and ventricular arrythmias, erectile dysfunctions, pulmonary diseases and asthma attacks, cyclic hormonal deficiencies and dysmenorrhea, incapacitating painful inflammatory attacks of Parkinson's disease.

## Patentansprüche

1. Galenische Form zur Verwendung als Medikament zur transmukosalen Verabreichung mindestens eines Wirkstoffes, **dadurch gekennzeichnet, dass** sie als Wirkstoff Sumatriptan, Loperamid, Cetirizin, Loratadin, Metopimazin, Domperidon, Dimenhydrinat, Diphenhydramin, Amiodaron, Flecainid, DHEA, Östradiol, Ibuprofen, Ketoprofen, Diclofenac, Sildenafil, Vardenafil, Tadalafil, Salbutamol, Salmeterol, (R)-Levosalbutamol, Apomorphin oder Selegilin umfasst, wobei der Wirkstoff als stabile und vollständige Auflösung in einer wässrigalkoholischen Lösung auf Basis von Wasser und Ethanol vorliegt, umfassend zwischen 20 % und 95 % Ethanol und zwischen 5 % und 80 % Wasser in Masse, so dass eine schnelle Aufnahme des Wirkstoffs über die sublingualen Schleimhäute oder die Schleimhäute des Zahnfleischs und/oder der Wangen ermöglicht wird.

2. Galenische Form nach Anspruch 1, wobei der mindestens eine Wirkstoff ein Molekulargewicht von weniger als 10 000 Da aufweist.

3. Galenische Form nach einem der vorhergehenden Ansprüche, wobei diese in einen flexiblen Behälter abgepackt wird, der die Stabilität des Wirkstoffs oder der Wirkstoffe in ihrer Auflösung im Laufe der Zeit gewährleistet.

4. Galenische Form nach Anspruch 3, wobei der Behälter in Form einer dichten Kapsel mit einem Fassungsvermögen von höchstens 5 ml für eine einzige Dose oder mehrere Dosen vorliegt.

5. Galenische Form nach einem der vorhergehenden Ansprüche, des Weiteren aufweisend ein pH-Ausgleichsmittel und/oder ein Sequestriermittel, wenn der Wirkstoff eine CarbonylFunktion umfasst.

6. Verwendung der galenischen Form nach einem der vorhergehenden Ansprüche 1 bis 5 zur Herstellung eines Medikamentes zur Behandlung und/oder Vorbeugung von Migräneanfällen, durchfallartigen Syndromen, allergischen Anfällen, Erbrechen, Symptomen der Übelkeit, supraventrikulären und ventrikulären Arrhythmien, Erektionsproblemen, Lungenleiden und Asthmaanfällen, zyklischen Hormoninsuffizienzen und Menstruationsbeschwerden, entzündlichen unangenehmen Schmerzanfällen, der Parkinson-Krankheit.
